(19) Europäisches Patentamt
European Patent Office
Office européen des brevets

(11) Veröffentlichungsnummer : **0 558 448 A1**

(12) # EUROPÄISCHE PATENTANMELDUNG

(21) Anmeldenummer : **93810095.5**

(22) Anmeldetag : **17.02.93**

(51) Int. Cl.$^5$ : **A01N 47/36,** // (A01N47/36, 43:56, 25:32)

(30) Priorität : **26.02.92 CH 583/92**
**31.08.92 CH 2727/92**

(43) Veröffentlichungstag der Anmeldung :
**01.09.93 Patentblatt 93/35**

(84) Benannte Vertragsstaaten :
**AT BE CH DE DK ES FR GB GR IE IT LI LU MC NL PT SE**

(71) Anmelder : **CIBA-GEIGY AG**
**Klybeckstrasse 141**
**CH-4002 Basel (CH)**

(72) Erfinder : **Glock, Jutta, Dr.**
**Innere Eigenmatt 3**
**CH-4336 Kaisten (CH)**
Erfinder : **Kerber, Elmar, Dr.**
**Niederwihl 131**
**NL-7883 Görwihl (DE)**

(54) **1,5-Diphenylpyrazol-3-Carbonsäure-Derivate als Antidots für Sulfonylharnstoffherbizide.**

(57) Ein selektiv-herbizides Mittel zur Bekämpfung von Gräsern und Unkräutern in Nutzpflanzenkulturen besteht

a) aus einer herbizid wirksamen Menge eines Sulfonylharnstoffs der Formel I

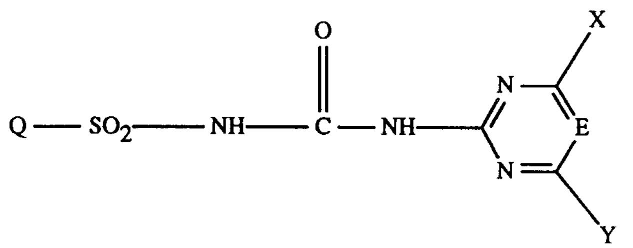

(I),

worin

Q einen Rest

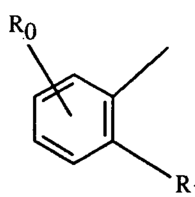

,

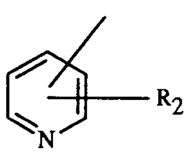

oder

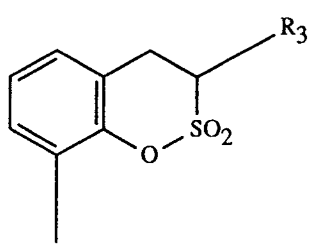

;

$R_0$ Wasserstoff, Halogen, Methyl oder Methoxy ;

$R_1$ Halogen, $C_1$-$C_3$-Halogenalkyl, $C_1$-$C_3$-Halogenalkoxy, $C_1$- oder $C_2$-Alkoxy-$C_1$-$C_3$-alkoxy, $C_3$-$C_6$-Alkenyloxy, $C_3$-$C_6$-Alkinyloxy, $C_2$- oder $C_3$-Alkinyl,

EP 0 558 448 A1

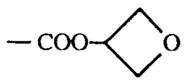

oder einen Rest

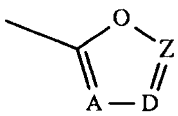

bedeutet ;
A und Z unabhängig voneinander Stickstoff oder Methin sind ;
D Stickstoff, Methin oder Methylmethin ;
$R_2$ $C_1$-$C_3$-Halogenalkoxy, $C_1$- oder $C_2$-Alkoxy-$C_1$-$C_3$-alkoxy, $C_4$-$C_6$-Cycloalkyloxy,$C_3$-$C_6$-Cycloalkyl-$C_1$-$C_3$-alkoxy, -$COOR_4$ oder -$NR_5R_5$ ist ;
$R_4$ $C_1$-$C_3$-Alkyl oder 3-Oxetanyl ;
$R_5$ Wasserstoff oder $C_1$-$C_4$-Alkyl ;
$R_5$ Wasserstoff, $C_1$-$C_4$-Alkyl oder $COR_{11}$ ;
$R_{11}$ Wasserstoff oder $C_1$-$C_6$-Alkyl ;
$R_3$ Wasserstoff oder Methyl ;
X Halogen, Methyl, Ethyl, Methoxy, Ethoxy, $C_1$- oder $C_2$-Halogenalkoxy, Cyclopropyl, -$NHCH_3$ oder -$N(CH_3)_2$ ;
Y Methyl, Ethyl, Methoxy, Ethoxy, $C_1$- oder $C_2$-Halogenalkoxy oder Cyclopropyl ; und
E Stickstoff oder die Methingruppe, bedeutet ;
sowie die agrochemisch verträglichen Salze dieser Verbindungen,
wobei D und Z nicht gleichzeitig Stickstoff sein dürfen ; und E die Methingruppe bedeutet, wenn X Halogen oder Difluormethoxy ist ; und

b) als Safener aus einer herbizid-antagonistisch wirksamen Menge eines 1,5-Diphenylpyrazol-3-carbonsäure-Derivates der Formel II

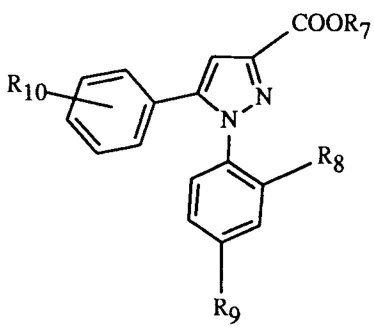
(II),

worin
$R_7$ Wasserstoff, $C_1$-$C_8$-Alkyl, $C_3$-$C_6$-Alkenyl, $C_3$-$C_6$-Cycloalkyl-$C_1$-$C_3$-alkyl, oder ein Alkalimetall oder Ammoniumkation ist ; und
$R_8$, $R_9$ und $R_{10}$ unabhängig voneinander Wasserstoff oder Halogen, bedeuten.

Die vorliegende Erfindung betrifft ein selektiv-herbizides Mittel zur Bekämpfung von Gräsern und Unkräutern in Kulturen von Nutzpflanzen, insbesondere in Getreidekulturen, welches aus einem Herbizid und einem Safener (Gegenmittel, Antidot) besteht, welches die Nutzpflanzen, nicht aber die Unkräuter vor der phytotoxischen Wirkung des Herbizides bewahrt, sowie die Verwendung dieses Mittels oder der Kombination Herbizid und Safener (Gegenmittel, Antidot) zur Unkrautbekämpfung in Nutzpflanzenkulturen.

Beim Einsatz von Herbiziden können in Abhängigkeit von Faktoren wie beispielsweise Dosis des Herbizids und Applikationsart, Art der Kulturpflanze, Bodenbeschaffenheit und klimatischen Bedingungen, wie beispielsweise Belichtungsdauer, Temperatur und Niederschlagsmengen, die Kulturpflanzen in erheblichem Maße geschädigt werden. Insbesondere kann es zu starken Schädigungen kommen, wenn im Rahmen der Fruchtfolge nach Kulturplanzen, die gegen die Herbizide resistent sind, andere Kulturplanzen angebaut werden, welche keine oder nur unzureichende Resistenz gegenüber den Herbiziden aufweisen.

Um diesem Problem zu begegnen, sind schon verschiedene Stoffe vorgeschlagen worden, welche befähigt sind, die schädigende Wirkung des Herbizids auf die Kulturpflanze spezifisch zu antagonisieren, das heißt, die Kulturpflanze zu schützen ohne dabei die Herbizidwirkung auf die zu bekämpfenden Unkräuter merklich zu beeinflussen. Dabei hat es sich gezeigt, daß die vorgeschlagenen Safener sowohl bezüglich der Kulturpflanzen als auch bezüglich des Herbizids und gegebenenfalls auch in Abhängigkeit von der Applikationsart oft sehr artspezifisch wirken, das heißt ein bestimmter Safener eignet sich oft nur für eine bestimmte Kulturpflanze und eine herbizide Stoffklasse. So sind beispielsweise aus EP-A-0 268 554 1,5-Diphenylpyrazol-3-carbonsäure-Derivate bekannt, die Kulturpflanzen vor der phytotoxischen Wirkung von Phenoxy-propionsäureester-Herbiziden schützen.

Es wurde nun gefunden, daß sich diese 1,5-Diphenylpyrazol-3-carbonsäure-Derivate zum Schützen von Kulturpflanzen vor der phytotoxischen Wirkung einer speziellen Klasse von Sulfonylharnstoffherbiziden eignen.

Erfindungsgemäß wird somit ein selektiv-herbizides Mittel vorgeschlagen, welches dadurch gekennzeichnet ist, daß es neben inerten Zutaten wie Trägerstoffen, Lösungsmitteln und Netzmitteln als wirksame Komponente eine Mischung enthält, die

a) aus einer herbizid wirksamen Menge eines Sulfonylharnstoffs der Formel I

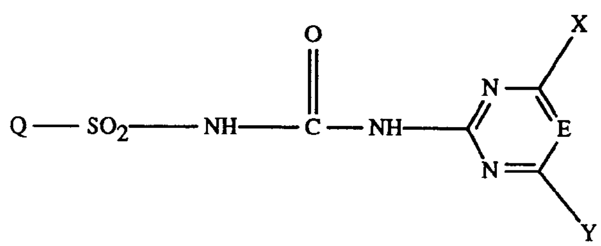

(I),

worin

Q einen Rest

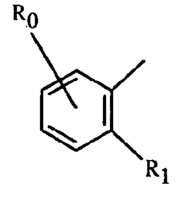

,

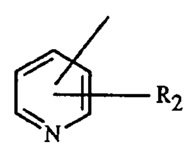

oder

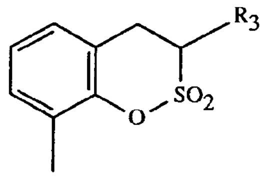

;

$R_0$ Wasserstoff, Halogen, Methyl oder Methoxy;

$R_1$ Halogen, $C_1$-$C_3$-Halogenalkyl, $C_1$-$C_3$-Halogenalkoxy, $C_1$- oder $C_2$-Alkoxy-$C_1$-$C_3$-alkoxy, $C_3$-$C_6$-Alkenyloxy, $C_3$-$C_6$-Alkinyloxy, $C_2$- oder $C_3$-Alkinyl,

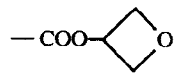

oder einen Rest

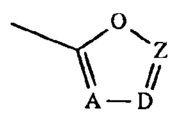

bedeutet;
A und Z unabhängig voneinander Stickstoff oder Methin sind;
D Stickstoff, Methin oder Methylmethin;
$R_2$ $C_1$-$C_3$-Halogenalkoxy, $C_1$- oder $C_2$-Alkoxy-$C_1$-$C_3$-alkoxy, $C_4$-$C_6$-Cycloalkyloxy, $C_3$-$C_6$-Cycloalkyl-$C_1$-$C_3$-alkoxy, -$COOR_4$ oder -$NR_5R_6$ ist;
$R_4$ $C_1$-$C_3$-Alkyl oder 3-Oxetanyl;
$R_5$ Wasserstoff oder $C_1$-$C_4$-Alkyl;
$R_6$ Wasserstoff, $C_1$-$C_4$-Alkyl oder -$COR_{11}$;
$R_{11}$ Wasserstoff oder $C_1$-$C_6$-Alkyl;
$R_3$ Wasserstoff oder Methyl;
X Halogen, Methyl, Ethyl, Methoxy, Ethoxy, $C_1$- oder $C_2$-Halogenalkoxy, Cyclopropyl, -$NHCH_3$ oder -$N(CH_3)_2$;
Y Methyl, Ethyl, Methoxy, Ethoxy, $C_1$- oder $C_2$-Halogenalkoxy oder Cyclopropyl; und
E Stickstoff oder die Methingruppe, bedeutet;
sowie die agrochemisch verträglichen Salze dieser Verbindungen, wobei D und Z nicht gleichzeitig Stickstoff sein dürfen; und E die Methingruppe bedeutet, wenn X Halogen oder Difluormethoxy ist; und
b) als Safener aus einer herbizid-antagonistisch wirksamen Menge eines 1,5-Diphenylpyrazol-3-carbonsäure-Derivates der Formel II

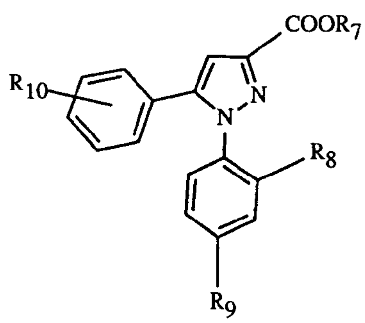

(II),

worin
$R_7$ Wasserstoff, $C_1$-$C_8$-Alkyl, $C_3$-$C_6$-Alkenyl, $C_3$-$C_6$-Cycloalkyl-$C_1$-$C_3$-alkyl, oder ein Alkalimetall oder Ammoniumkation ist; und
$R_8$, $R_9$ und $R_{10}$ unabhängig voneinander Wasserstoff oder Halogen bedeuten, besteht.

In den Verbindungen der Formel I und II bedeutet Halogen Fluor, Chlor, Brom oder Jod; vorzugsweise Fluor, Chlor oder Brom, insbesondere Fluor und Chlor.

Als Alkylgruppen kommen geradkettige oder verzweigte Alkylgruppen in Betracht, beispielsweise Methyl, Ethyl, n-Propyl, iso-Propyl, n-Butyl, iso-Butyl, sek.-Butyl oder tert.-Butyl, sowie die verschiedenen isomeren Pentyl-, Hexyl-, Heptyl- und Octyl-Reste.

Als Halogenalkyl kommen ein- oder mehrfach durch Halogen substituierte Alkylgruppen in Betracht, wobei Halogen im einzelnen Fluor, Chlor, Brom oder Jod bedeutet. Bevorzugt sind unter diesen ein- oder mehrfach durch Halogen substituierten Alkylgruppen ein- bis dreifach durch Halogen, insbesondere Fluor, Chlor oder Brom substituierte Alkylgruppen, beispielsweise Fluormethyl, Difluormethyl, Trifluormethyl, Chlormethyl, Dichlormethyl, Trichlormethyl, Difluor-chlormethyl, 2-Fluorethyl, 2-Chlorethyl, 2,2,2-Triluorethyl, 2,2,2-Trichlorethyl; vorzugsweise 2-Fluor-n-propyl und 3-Fluor-n-propyl.

Alkoxyalkoxy ist z.B. Methoxymethoxy, Ethoxymethoxy, Methoxyethoxy, Ethoxyethoxy, Methoxypropyloxy oder Ethoxypropyloxy.

Halogenalkoxy ist z.B. Fluormethoxy, Difluormethoxy, Trifluormethoxy, 2,2,2-Trifluorethoxy, 1,1,2,2-Tetrafluorethoxy, 2-Fluorethoxy, 2-Chlorethoxy oder 2,2,2-Trichlorethoxy; vorzugsweise Difluormethoxy, Trifluormethoxy und 2-Chlorethoxy.

Als Alkenylgruppen kommen geradkettige oder verzweigte Alkenylgruppen in Betracht wie z.B. Allyl, Methallyl, But-2-en-1-yl, 3-Pentenyl oder 2-Hexenyl.

Im Rahmen der vorliegenden Erfindung sind die an Sauerstoff gebundenen Alkenyl- und Alkinylgruppen in der Regel über ein gesättigtes Kohlenstoffatom gebunden.

Als Alkenyloxygruppen kommen geradkettige oder verzweigte Alkenyloxygruppen in Betracht wie beispielsweise Allyloxy, Methallyloxy, But-2-en-1-yloxy, 3-Pentenyloxy oder 2-Hexenyloxy; vorzugsweise Allyloxy.

Als Alkinyloxygruppen kommen geradkettige oder verzweigte Alkinyloxygruppen in Betracht wie beispielsweise Propargyloxy, 1-Methylpropargyloxy, 3-Butinyloxy, 2-Pentinyloxy oder 2-Hexinyloxy. Bevorzugt ist Propargyloxy.

Als Cycloalkyloxy kommen beispielsweise Cyclobutyloxy, Cyclopentyloxy oder Cyclohexyloxy in Betracht; vorzugsweise Cyclopentyloxy und Cyclohexyloxy.

Als Cycloalkyl-alkoxygruppen kommen beispielsweise Cyclopropyl-methyloxy, Cyclobutyl-methyloxy, Cyclopentyl-methyloxy, Cyclohexyl-methyloxy, Cyclopropyl-ethyloxy, Cyclopentyl-ethyloxy oder Cyclohexyl-ethyloxy in Betracht; vorzugsweise Cyclopropyl-methyloxy.

Als Cycloalkyl-substituiertes Alkyl kommen beispielsweise Cyclopropyl-methyl, Cyclobutyl-methyl, Cyclopentyl-methyl, Cyclohexyl-methyl, Cyclopropyl-ethyl oder Cyclohexyl-ethyl in Betracht; vorzugsweise Cyclohexyl-methyl.

Die Verbindungen der Formel I und II können Salze bilden, bei denen der Wasserstoff der $-SO_2$-NH-Gruppe in der Verbindung der Formel I bzw. der Wasserstoff des Restes $R_7$ in der Verbindung der Formel II durch ein für die Landwirtschaft geeignetes Kation ersetzt wird. Diese Salze sind beispielsweise Metallsalze, insbesondere Alkalisalze für die Verbindungen der Formel II ($R_7$ Wasserstoff) oder Alkali- oder Erdalkalisalze für die Verbindungen der Formel I, oder auch Ammoniumsalze oder Salze mit organischen Aminen. Ebenso kann Salzbildung durch Anlagerung einer starken Säure an den Pyrimidin- oder Triazinteil der Verbindungen der Formel I, und an den Pyrazolteil der Verbindungen der Formel II erfolgen. Geeignete Säuren hierfür sind Salzsäure, Bromwasserstoffsäure, Schwefelsäure oder Salpetersäure.

Als Beispiele für zur Ammonium-Kationenbildung geeignete Amine kommen sowohl Ammoniak wie auch primäre, sekundäre und tertiäre $C_1$-$C_4$-Alkylamine, $C_1$-$C_4$-Hydroxyalkylamine und $C_2$-$C_4$-Alkoxyalkylamine in Betracht, beispielsweise Methylamin, Ethylamin, n-Propylamin, iso-Propylamin, die vier isomeren Butylamine, n-Amylamin, iso-Amylamin, Hexylamin, Heptylamin, Octylamin, Nonylamin, Decylamin, Pentadecylamin, Hexadecylamin, Heptadecylamin, Octadecylamin, Methyl-ethylamin, Methyl-iso-propylamin, Methyl-hexylamin, Methyl-nonylamin, Methyl-pentadecylamin, Methyl-octadecylamin, Ethyl-butylamin, Ethyl-heptylamin, Ethyl-octylamin, Hexyl-heptylamin, Hexyl-octylamin, Dimethylamin, Diethylamin, Di-n-propylamin, Di-iso-propylamin, Di-n-butylamin, Di-n-amylamin, Di-iso-amylamin, Dihexylamin, Diheptylamin, Dioctylamin, Ethanolamin, n-Propanolamin, iso-Propanolamin, N,N-Diethanolamin, N-Ethylpropanolamin, N-Butylethanolamin, Allylamin, n-Butenyl-2-amin, n-Pentenyl-2-amin, 2,3-Dimethylbutenyl-2-amin, Di-butenyl-2-amin, n-Hexenyl-2-amin, Propylendiamin, Diethanolamin, Trimethylamin, Triethylamin, Tri-n-propylamin, Tri-iso-propylamin, Tri-n-butylamin, Tri-iso-butylamin, Tri-sek.-butylamin, Tri-n-amylamin, Methoxyethylamin und Ethoxyethylamin; heterocyclische Amine wie z.B. Pyridin, Chinolin, iso-Chinolin, Morpholin, Piperidin, Pyrrolidin, Indolin, Chinuclidin und Azepin; primäre Arylamine wie z.B. Aniline, Methoxyaniline, Ethoxyaniline, o,m,p-Toluidine, Phenylendiamine, Benzidine, Naphthylamine und o,m,p-Chloraniline; insbesondere aber Triethylamin, iso-Propylamin und Di-iso-propylamin.

Für die Verwendung im erfindungsgemässen Mittel bevorzugte Verbindungen der Formel I oder deren Salze sind solche der Formel Ia

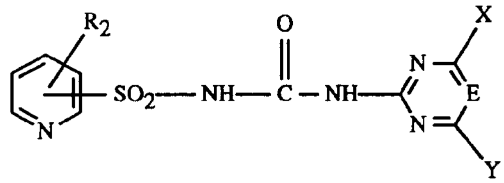

(Ia),

worin $R_2$, X, Y und E die unter Formel I angegebenen Bedeutungen haben; insbesondere diejenigen der Formel Ib

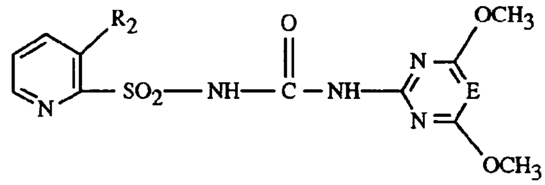

(Ib),

worin $R_2$ und E die unter Formel I angegebenen Bedeutungen haben.

Für die Verwendung im erfindungsgemässen Mittel ebenfalls bevorzugte Verbindungen der Formel I oder deren Salze sind solche der Formel Ic

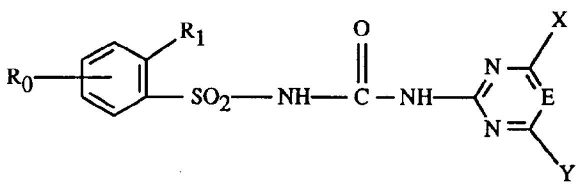

(Ic),

worin $R_0$, $R_1$, X, Y und E die unter Formel I angegebenen Bedeutungen haben; insbesondere diejenigen der Formel Id

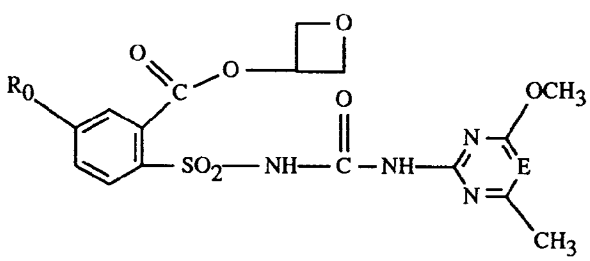

(Id),

worin $R_0$ Wasserstoff oder Fluor, und E die Methingruppe oder Stickstoff, bedeutet.

Für die Verwendung im erfindungsgemässen Mittel ganz besonders bevorzugte Verbindungen der Formel I oder deren Salze sind solche, worin Q einen Rest

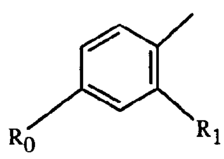

,

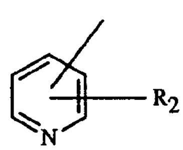

oder

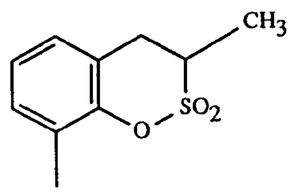 ;

$R_0$ Wasserstoff oder Halogen, insbesondere Fluor;
$R_1$ $C_1$-$C_3$-Monofluoralkyl, Difluormethoxy, Propargyloxy, Ethinyl,

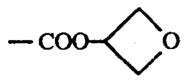

oder
einen Rest

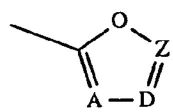

bedeutet; A und Z unabhängig voneinander Stickstoff oder Methin sind; D Stickstoff, Methin oder Methylmethin; $R_2$ in 2- oder 3-Position, alternierend mit der Position der -$SO_2$NHCO-Gruppe, $C_1$- oder $C_2$-Halogenalkoxy, Cyclopropylmethylenoxy, $COOR_4$ oder $NR_0R_6$; $R_4$ Methyl oder 3-Oxetanyl; $R_0$ Wasserstoff, Methyl oder Ethyl; $R_0$ Wasserstoff, Methyl, Ethyl, -CHO, -$COCH_3$ oder -$COC_2H_5$ ist; X und Y unabhängig voneinander Methyl, Methoxy, Ethoxy oder Cyclopropyl sind; und E Stickstoff oder die Methingruppe ist.

Bevorzugte Verbindungen der Formel II für die Verwendung im erfindungsgemässen Mittel, insbesondere bei denjenigen Mitteln, die die Verbindungen der Formel I mit den oben als bevorzugt und besonders bevorzugt genannten Substituenten enthalten, sind 1,5-Diphenylpyrazol-3-carbonsäure-Derivate der Formel IIa

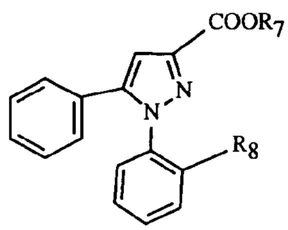 (IIa),

worin $R_7$ und $R_0$ die unter Formel II angegebenen Bedeutungen haben.

Besonders bevorzugte Verbindungen der Formel IIa sind diejenigen der Formel IIb

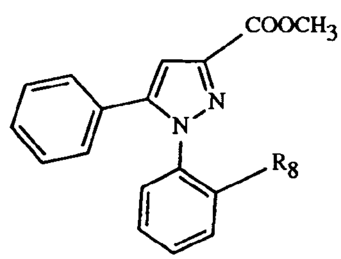 (IIb),

worin $R_0$ die unter Formel II angegebene Bedeutung hat.

Ganz besonders bevorzugte Verbindungen der Formel II sind solche, worin $R_7$ Wasserstoff, $C_1$-$C_8$-Alkyl, Methallyl, Cyclohexyl-methylenyl, Natrium, Triethylammonium oder iso-Propylammonium; $R_8$ Wasserstoff oder Halogen; $R_9$

Wasserstoff oder Chlor, und $R_{10}$ Wasserstoff, Fluor oder Chlor, bedeuten.

Als ganz besonders bevorzugte Einzelverbindung aus dem Umfang der Formel II gilt diejenige der Formel IIc

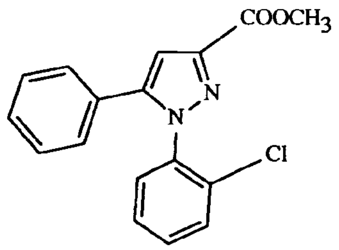

(IIc).

Ein ganz besonders bevorzugtes erfindungsgemässes Mittel enthält als Herbizid der Formel I N-[2-((3-Oxetanyl)-oxycarbonyl)-phenylsulfonyl]-N'-(4-methyl-6-methoxypyrimidin-2-yl)-harnstoff (Verbindung der Formel Id, worin $R_0$ Wasserstoff und E Methin bedeutet) und als Safener eine Verbindung ausgewählt aus:
1-(2-Chlorphenyl)-3-methoxycarbonyl-5-phenylpyrazol;
1-(2,4-Dichlorphenyl)-3-methoxycarbonyl-5-phenylpyrazol;
1-(2-Chlorphenyl)-3-benzyloxycarbonyl-5-phenylpyrazol;
1-(2-Chlorphenyl)-3-methoxycarbonyl-5-(2,4-dichlorphenyl)-pyrazol; oder
1-(2-Chlorphenyl)-3-methoxycarbonyl-5-(2-fluorphenyl)-pyrazol.
Ein ganz besonders bevorzugtes erfindungsgemässes Mittel enthält als Safener 1-(2-Chlorphenyl)-3-methoxycarbonyl-5-phenylpyrazol und als Herbizid aus dem Umfang der Formel I:
N-[2-(Cyclopropylmethylenoxy)-3-pyridylsulfonyl]-N'-(4,6-dimethoxypyrimidin-2-yl-)harnstoff;
N-[3-(Cyclopropylmethylenoxy)-2-pyridylsulfonyl]-N'-(4,6-dimethoxypyrimidin-2-yl)-harnstoff;
N-[3-Dimethylamino-2-pyridylsulfonyl]-N'-(4,6-dimethoxypyrimidin-2-yl)-harnstoff;
N-[2-Dimethylamino-3-pyridylsulfonyl]-N'-(4-methyl-6-methoxypyrimidin-2-yl)-harnstoff:
N-[3-Dimethylamino-2-pyridylsulfonyl]-N'-(4-methyl-6-methoxypyrimidin-2-yl)-harnstoff;
N- [2-((3-Oxetanyl)-oxycarbonyl)-phenylsulfonyl]-N'-(4,6-dimethylpyrimidin-2-yl)-harnstoff;
N- [2-((3-Oxetanyl)-oxycarbonyl)-phenylsulfonyl]-N'-(4-methyl-6-methoxypyrimidin-2-yl)-harnstoff;
N-[2-((3-Oxetanyl)-oxycarbonyl)-phenylsulfonyl]-N'-(4,6-dimethoxypyrimidin-2-yl)-harnstoff;
N-[3-Difluormethoxy-2-pyridylsulfonyl]-N'-(4-methyl-6-methoxypyrimidin-2-yl)-harnstoff;
N- [3-((3-Oxetanyl)-oxycarbonyl)-2-pyridylsulfonyl]-N'-(4,6-dimethoxypyrimidin-2-yl)-harnstoff; oder
N-[2-((3-Oxetanyl)-oxycarbonyl)-4-fluor-phenylsulfonyl]-N'-(4-methyl-6-methoxypyrimidin-2-yl)-harnstoff.

Die Herstellung der Pyridyl-sulfonylharnstoffe der Formel Ia'

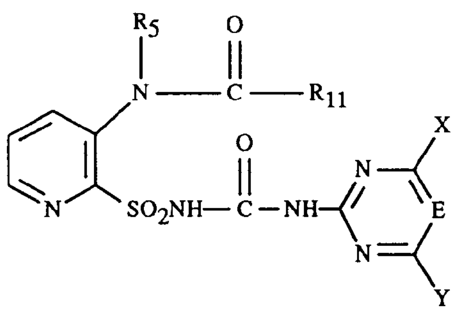

(Ia'),

worin $R_5$, $R_{11}$, X, Y und E die unter Formel I angegebenen Bedeutungen haben, erfolgt in Analogie zu bekannten Verfahren und ist dadurch gekennzeichnet, dass man ein Pyridylsulfonamid der Formel III

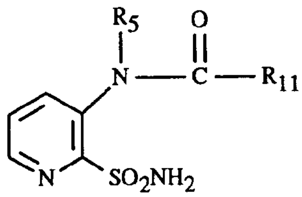

(III),

worin $R_5$ und $R_{11}$ die unter Formel I angegebene Bedeutung haben, mit einem N-Pyrimidinylcarbamat der Formel IV

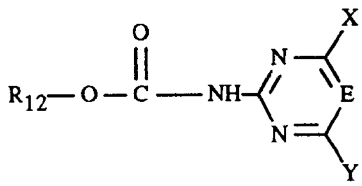

(IV),

worin X, Y und E die unter Formel I angegebene Bedeutung haben und $R_{12}$ $C_1$-$C_4$-Alkyl oder Phenyl, welches durch $C_1$-$C_4$-Alkyl oder Halogen substituiert sein kann, bedeutet, in Gegenwart einer Base umsetzt.

Die Umsetzungen zu Verbindungen der Formel Ia' werden vorteilhafterweise in aprotischen, inerten organischen Lösungsmitteln vorgenommen. Solche Lösungsmittel sind Kohlenwasserstoffe wie Benzol, Toluol, Xylole oder Cyclohexan, chlorierte Kohlenwasserstoffe wie Dichlormethan, Trichlormethan, Tetrachlormethan oder Chlorbenzol, Ether wie Diethylether, Ethylenglykoldimethylether, Diethylenglykoldimethylether, Tetrahydrofuran oder Dioxan, Nitrile wie Acetonitril oder Propionitril, Amide wie Dimethylformamid, Diethylformamid oder N-Methylpyrrolidinon. Die Reaktionstemperaturen liegen vorzugsweise zwischen -20° und + 120°C.

Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triethylamin, Chinuclidin, 1,4-Diazabicyclo[2.2.2]octan, 1,5-Diazabicyclo[4.3.0]non-5-en oder 1,5-Diazabicyclo[5.4.0]undec-7-en geeignet. Als Basen können aber auch anorganische Basen wie Hydride, z.B. Natrium- oder Calciumhydrid; Hydroxide, z.B. Natrium- und Kaliumhydroxid; Carbonate, z.B. Natrium- und Kaliumcarbonat; oder Hydrogencarbonate, z.B. Kalium- und Natriumhydrogencarbonat verwendet werden.

Die Endprodukte der Formel Ia' können durch Einengen und/oder Verdampfen des Lösungsmittels isoliert und durch Umkristallisieren oder Zerreiben des festen Rückstandes in Lösungsmitteln, in denen sie sich nicht gut lösen, wie Ether, aromatischen Kohlenwasserstoffen oder chlorierten Kohlenwasserstoffen, gereinigt werden.

Die Zwischenprodukte der Formel III und IV sind bekannt und können analog zu bekannten Verfahren hergestellt werden. Verfahren zur Herstellung von N-Pyrimidinylcarbamaten der Formel IV sind beispielsweise in EP-A-0 101 670 beschrieben.

Verbindungen der Formel III können analog den in der EP-A-0 314 505 und EP-A-0 459 949 beschriebenen Verfahren hergestellt werden.

Beispielsweise kann man eine Verbindung der Formel V

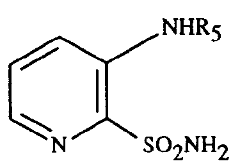

(V),

worin $R_5$ die unter Formel I angegebene Bedeutung hat, mit dem Acylierungsmittel der Formel VI

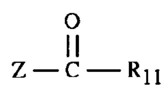

(VI),

worin $R_{11}$ die unter Formel I angegebene Bedeutung hat und Z Halogen, $R_{11}O$- oder eine übliche Abgangsgruppe bedeutet, in Gegenwart einer Base in die Verbindung der Formel III überführen. Derartige Umsetzungen sind beispielsweise in Farmaco Ed. scient. 12, 392 (1957) beschrieben. Die Verbindungen der Formel V sind bekannt und können nach bekannten Methoden, die beispielsweise in der EP-A-0 459 949 offenbart sind, hergestellt werden.

Die Sulfonylharnstoffherbizide der Formel I sind bekannt und ihre Herstellung ist z.B. in US-A-4 544 401, US-A-4 618 363, EP-A-0 044 807, EP-A-0 099 339, EP-A-0 102 925, EP-A-0 103 543, EP-A-0 120 814, EP-A-0 145 664 und EP-A-0 459 949 beschrieben.

Die 1,5-Diphenylpyrazol-3-carbonsäure-Derivate der Formel II sind bekannt und ihre Herstellung ist z.B. in EP-A-0 268 554 beschrieben.

Für die erfindungsgemäße Verwendung besonders gut geeignete Herbizide der Formel I sind in den nachfolgenden Tabellen 1, 2 und 3 beschrieben.

Tabelle 1: Verbindungen der Formel Ic

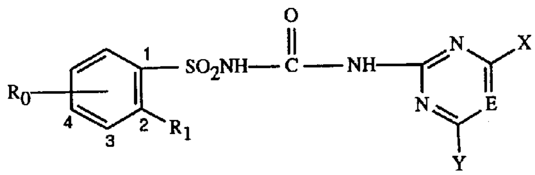

(Ic)

| Verb.Nr. | $R_1$ | $R_0$ | X | Y | E |
|---|---|---|---|---|---|
| 1.01 | $-OCHF_2$ | H | $-OCH_3$ | $-OCH_3$ | CH |
| 1.02 | 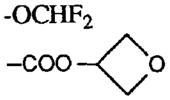 | H | $-CH_3$ | $-OCH_3$ | CH |
| 1.03 | 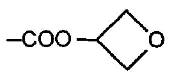 | H | $-CH_3$ | $-CH_3$ | CH |
| 1.04 | 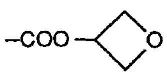 | H | $-OCH_3$ | $-OCH_3$ | CH |
| 1.05 | $-COOCH_3$ | H | $-OC_2H_5$ | 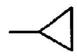 | N |
| 1.06 | $-OCH_2C{\equiv}CH$ | H | $-OCH_3$ | $-CH_3$ | N |
| 1.07 | $-CH_2CH_2CH_2-F$ | H | $-OCH_3$ | $-CH_3$ | CH |
| 1.08 | $-CH_2CH_2-F$ | H | $-OCH_3$ | $-CH_3$ | N |
| 1.09 | 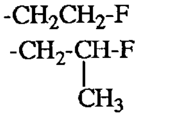 | H | $-OCH_3$ | $-CH_3$ | N |
| 1.10 | $-C{\equiv}CH$ | H | $-OCH_3$ | $-CH_3$ | N |
| 1.11 | $-C{\equiv}CH$ | H | $-OCH_3$ | $-CH_3$ | CH |
| 1.12 | $-C{\equiv}CH$ | H | $-OCH_3$ | $-OCH_3$ | CH |
| 1.13 | 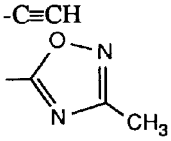 | H | $-OCH_3$ | $-OCH_3$ | CH |
| 1.14 | 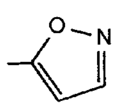 | H | $-OCH_3$ | $-OCH_3$ | CH |

Tabelle 1: (Forts.)

| Verb.Nr. | $R_1$ | $R_0$ | X | Y | E |
|---|---|---|---|---|---|
| 1.15 | 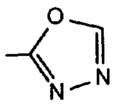 | H | $-OCH_3$ | $-OCH_3$ | CH |
| 1.16 | 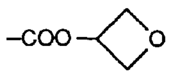 | 4-F | $-OCH_3$ | $-CH_3$ | CH |

Tabelle 2: Verbindungen der Formel Ia

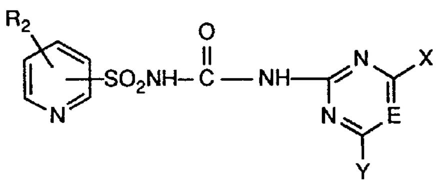

(Ia)

| Verb.Nr. | Position $-SO_2NHCO-$ | $R_2$ | X | Y | E |
|---|---|---|---|---|---|
| 2.01 | 3 | $2-N(CH_3)_2$ | $-OCH_3$ | $-OCH_3$ | CH |
| 2.02 | 3 | 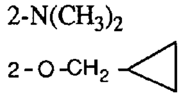 | $-OCH_3$ | $-OCH_3$ | CH |
| 2.03 | 2 | 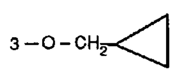 | $-OCH_3$ | $-OCH_3$ | CH |
| 2.04 | 2 | $3-OCHF_2$ | $-OCH_3$ | $-OCH_3$ | CH |
| 2.05 | 2 | $3-OCHF_2$ | $-OCH_3$ | $-OCH_3$ | CH |
| 2.06 | 2 | $3-N(CH_3)_2$ | $-OCH_3$ | $-OCH_3$ | CH |
| 2.07 | 2 | $3-NHC_2H_5$ | $-OCH_3$ | $-OCH_3$ | CH |
| 2.08 | 2 | $3-N(CH_3)_2$ | $-OCH_3$ | $-CH_3$ | CH |
| 2.09 | 2 | 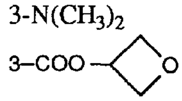 | $-OCH_3$ | $-OCH_3$ | CH |
| 2.10 | 2 | $3-OCH_2CH_2Cl$ | $-OCH_3$ | $-OCH_3$ | CH |

Tabelle 3: Verbindungen der Formel

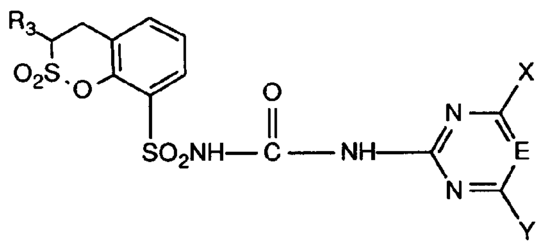

| Verb.Nr. | $R_3$ | X | Y | E |
|---|---|---|---|---|
| 3.01 | $-CH_3$ | $-CH_3$ | $-CH_3$ | CH |

Für die erfindungsgemässe Verwendung besonders gut geeignete Safener der Formel II sind in der nachfolgenden Tabelle 4 aufgeführt.

Tabelle 4: Verbindungen der Formel II

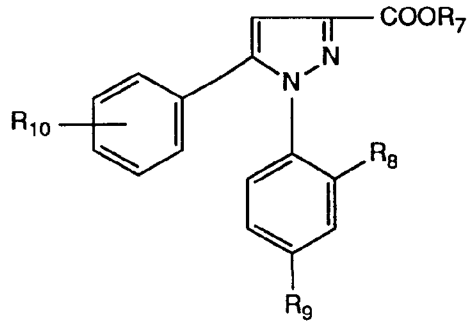

(II)

| Verb.Nr. | $R_8$ | $R_9$ | $R_{10}$ | $R_7$ |
|---|---|---|---|---|
| 4.01 | Cl | H | H | $-CH_3$ |
| 4.02 | Cl | H | H | H |
| 4.03 | F | H | H | $-CH_3$ |
| 4.04 | Cl | H | 3-F | $-CH_3$ |
| 4.05 | Br | H | H | $-CH_3$ |
| 4.06 | H | H | H | $-CH_3$ |
| 4.07 | Cl | H | H | $-CH_2-C(CH_3)=CH_2$ |
| 4.08 | Cl | H | H | $-C_4H_9(n)$ |
| 4.09 | Cl | H | H | $-CH(CH_3)(C_5H_{11}-n)$ |
| 4.10 | Cl | H | H | $-C_8H_{17}(n)$ |
| 4.11 | H | H | H | $-C_2H_5$ |
| 4.12 | Cl | Cl | H | $-CH_3$ |
| 4.13 | Cl | H | 3-Cl | $-CH_3$ |
| 4.14 | Cl | H | H | $HN^{\oplus}(C_2H_5)_3$ |
| 4.15 | Cl | H | H | $H_3N^{\oplus}-CH(CH_3)_2$ |
| 4.16 | Cl | H | H | $Na^{\oplus}$ |
| 4.17 | Cl | H | H | 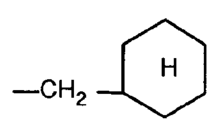 |
| 4.18 | Cl | Cl | 2-Cl | $-CH_3$ |
| 4.19 | Cl | H | 2-F | $-CH_3$ |

Die Erfindung betrifft auch ein Verfahren zum selektiven Bekämpfen von Unkräutern in Nutzpflanzenkulturen, welches darin besteht, daß man die Nutzpflanzen, deren Samen oder Stecklinge oder deren Anbaufläche gleichzeitig oder unabhängig voneinander mit einer herbizid wirksamen Menge des Sulfonylharnstoffs der Formel I und einer herbizid-antagonistisch wirksamen Menge eines 1,5-Diphenylpyrazol-3-carbonsäure-Derivates der Formel II behandelt.

Als Kulturpflanzen, welche durch die 1,5-Diphenylpyrazol-3-carbonsäure-Derivate der Formel II gegen schädigende Wirkung der oben erwähnten Herbizide geschützt werden können, kommen insbesondere diejenigen in Betracht, die auf dem Nahrungs- oder Textilsektor von Bedeutung sind, beispielsweise Zuckerrohr und insbesondere Kulturhirse, Mais, Reis und andere Getreidearten (Weizen, Roggen, Gerste, Hafer), ganz besonders aber Weizen und Gerste.

Bei den zu bekämpfenden Unkräutern kann es sich sowohl um monokotyle wie um dikotyle Unkräuter handeln.

Als Kulturpflanzen oder Teile dieser Pflanzen kommen beispielsweise die vorstehend genannten in Betracht. Als Anbauflächen gelten die bereits mit den Kulturpflanzen bewachsenen oder mit dem Saatgut dieser Kulturpflanzen beschickten Bodenareale, wie auch die zur Bebauung mit diesen Kulturpflanzen bestimmten Böden.

Ein Safener oder Antidot der Formel II kann je nach Anwendungszweck zur Vorbehandlung des Saatgutes der Kulturpflanze (Beizung des Samens oder der Stecklinge) eingesetzt oder vor oder nach der Saat in den Boden gegeben werden. Er kann aber auch für sich allein oder zusammen mit dem Herbizid vor oder nach dem Auflaufen der Pflanzen appliziert werden. Die Behandlung der Pflanze oder des Saatgutes mit dem Safener kann daher grundsätzlich unabhängig vom Zeitpunkt der Applikation der phytotoxischen Chemikalie erfolgen. Die Behandlung der Pflanze kann jedoch auch durch gleichzeitige Applikation von phytotoxischer Chemikalie und Safener (Tankmischung) erfolgen. Die pre-emergente Behandlung schließt sowohl die Behandlung der Anbaufläche vor der Aussaat (ppi = pre plant incorporation) als auch die Behandlung der angesäten, aber noch nicht bewachsenen Anbauflächen ein.

Die zu applizierende Aufwandmenge Safener im Verhältnis zum Herbizid richtet sich weitgehend nach der Anwendungsart. Bei einer Feldbehandlung, welche entweder unter Verwendung einer Tankmischung mit einer Kombination von Safener und Herbizid oder durch getrennte Applikation von Safener und Herbizid erfolgt, liegt in der Regel ein Verhältnis von Safener zu Herbizid von 1:100 bis 10:1, bevorzugt 1:20 bis 1:1, und insbesondere 1:1, vor. Dagegen werden bei der Samenbeizung weit geringere Mengen Safener im Verhältnis zur Aufwandmenge an Herbizid pro Hektar Anbaufläche benötigt.

In der Regel werden bei der Feldbehandlung 0,001 bis 5,0 kg Safener/ha, vorzugsweise 0,01 bis 0,5 kg Safener/ha, appliziert.

Die Aufwandmengen an Herbizid liegt in der Regel zwischen 0,001 bis 4 kg/ha, vorzugsweise jedoch zwischen 0,05 bis 2 kg/ha.

Bei der Samenbeizung werden im allgemeinen 0,001 bis 10 g Safener/kg Samen, vorzugsweise 0,05 bis 2 g Safener/kg Samen, appliziert. Wird der Safener in flüssiger Form kurz vor der Aussaat unter Samenquellung appliziert, so werden zweckmäßigerweise Safener-Lösungen verwendet, welche den Wirkstoff in einer Konzentration von 1 bis 10000, vorzugsweise von 100 bis 1000 ppm, enthalten.

Zur Applikation werden die Verbindungen der Formel II oder Kombinationen von Verbindungen der Formel II mit den zu antagonisierenden Herbiziden zweckmäßigerweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen, Bestreichen oder Gießen werden gleich wie die Art der zu verwendenden Mittel den angestrebten Zielen und den gegebenen Verhältnissen entsprechend gewählt.

Die Formulierungen, d.h. die den Wirkstoff der Formel II oder eine Kombination von Wirkstoff der Formel II mit zu antagonisierendem Herbizid der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen un/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Frakrionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole, sowie deren Ether und Ester wie Ethanol, Ethylenglykol, Ethylenglykolmonomethyl- oder ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnußöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als obeflächenaktive Verbindungen kommen je nach Art des zu formulierenden Wirkstoffs der Formel II und gegebenenfalls auch dem zu antagonisierenden Herbizid der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuß- oder Talgöl gewonnen werden können, genannt. Ferner sind auch die Fettsäure-methyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte and Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood New Jersey, 1981. Stache, H., "Tensid-Taschenbuch", Carl Hanser Verlag, München/Wien, 1981.

Die agrochemischen Zubereitungen enthalten in der Regel 0,1 bis 99 Gewichtsprozent, insbesondere 0,1 bis 95 Gew.-%, Wirkstoff der Formel II oder Wirkstoffgemisch Antidot/Herbizid, 1 bis 99,9 Gew.-%, insbesondere 5 bis 99,8 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Für die Verwendung von Verbindungen der Formel II oder sie enthaltender Mittel zum Schützen von Kulturpflanzen gegen schädigende Wirkungen von Herbiziden der Formel I kommen verschiedene Methoden und Techniken in Betracht, wie beispielsweise die folgenden:

i) Samenbeizung

a) Beizung der Samen mit einem als Spritzpulver formulierten Wirkstoff der Formel II durch Schütteln in einem Gefäß bis zur gleichmäßigen Verteilung auf der Samenoberfläche (Trockenbeizung). Man verwendet dabei etwa 1 bis 500 g Wirkstoff der Formel II (4 g bis 2 kg Spritzpulver) pro 100 kg Saatgut.

b) Beizung der Samen mit einem Emulsionskonzentrat des Wirkstoffs der Formel II nach der Methode a) (Naßbeizung).

c) Beizung durch Tauchen des Saatguts in eine Brühe mit 100-1000 ppm Wirkstoff der Formel II während I bis 72 Stunden und gegebenenfalls nachfolgendes Trocknen der Samen (Tauchbeizung).

Die Beizung des Saatguts oder die Behandlung des angekeimten Sämlings sind naturgemäß die bevorzugten Methoden der Applikation, weil die Wirkstoffbehandlung vollständig auf die Zielkultur gerichtet ist. Man verwendet in der Regel 1 bis 1000 g Antidot, vorzugsweise 5 bis 250 g Antidot, pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Applikation aus Tankmischung

Eine flüssige Aufarbeitung eines Gemisches von Antidot und Herbizid (gegenseitiges Mengenverhältnis zwischen 10:1 und 1:100) wird verwendet, wobei die Aufwandmenge an Herbizid 0,01 bis 5,0 kg pro Hektar beträgt. Solche Tankmischung wird vor oder nach der Aussaat appliziert.

iii) Applikation in der Saatfurche

Das Antidot wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

iv) Kontrollierte Wirkstoffabgabe

Der Wirkstoff der Formel II wird in Lösung auf mineralische Granulatträger oder polymerisierte Granulate (Harnstoff/Formaldehyd) aufgezogen und trocknen gelassen. Gegebenenfalls kann ein Überzug aufgebracht werden (Umhüllungsgranulate), der es erlaubt, den Wirkstoff über einen bestimmten Zeitraum dosiert abzugeben.

Herstellungsbeispiele

Beispiel H1: Herstellung von 3-(N-Methyl-N-acetylamino)-pyridin-2-ylsulfonamid

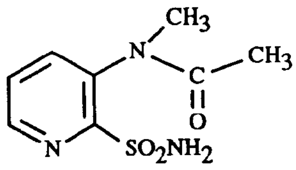

Zu einer Lösung von 1,87 g 3-N-Methylaminopyridin-2-ylsulfonamid in 40 ml trockenem Acetonitril gibt man bei Raumtemperatur nacheinander 0,89 ml Pyridin und 0,82 ml Acetylbromid hinzu. Nach 30 Minuten gibt man weitere 0,3 ml Acetylbromid, und nach einer weiteren Stunde Rühren 0,32 ml Pyridin hinzu. Nach 30-minütigem Rühren wird das Produkt 3-(N-Methyl-N-acetylamino)-pyridin-2-ylsulfonamid aus der Reaktionsmischung abfiltriert; Smp. 181-185°C.

Beispiel H2: Herstellung von N-(N-Methyl-N-acetylamino)-pyridin-2-ylsulfonyl-N'-(4-methoxy-6-methylpyrimidin-2-yl)-harnstoff

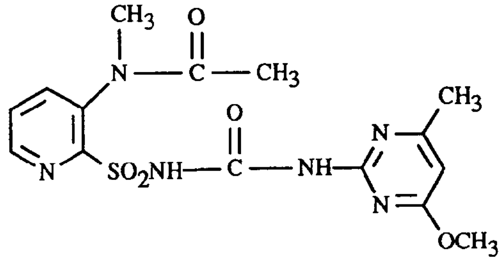

Zu einer Lösung von 3,07 g 3-(N-Methyl-N-acetylamino)-pyridin-2-ylsulfonamid in 50 ml Acetonitril gibt man nacheinander 2,18 ml 1,5-Diazabicyclo[5.4.0]undec-5-en und 3,89 g N-(4-Methoxy-6-methylpyrimidin-2-yl)-phenylcarbamat hinzu. Nach 45-minütigem Rühren wird die Reaktionsmischung im Vakuum eingeengt, der ölige Rückstand mit 10 ml 2N Salzsäure verrieben und anschließend mit 10 ml Wasser verdünnt. Das kristalline Produkt wird abfiltriert und anschließend mit Wasser und Diethylether gewaschen. Man erhält 5,25 g N-(N-Methyl-N-acetylamino)-pyridin-2-ylsulfonyl-N'-(4-methoxy-6-methylpyrimidin-2-yl)-harnstoff mit einem Schmelzpunkt von 178-180°C.

Formulierunpsbeispiele für flüssige Wirkstoffe der Formel II oder Mischungen derselben mit einem Herbizid der Formel I (% = Gewichtsprozent)

| 1. Emulsions-Konzentrate | a) | b) | c) |
|---|---|---|---|
| Wirkstoffmischung | 25 % | 40 % | 50 % |
| Ca-Dodecylbenzolsulfonat | 5 % | 8 % | 6 % |
| Ricinusöl-polyethylenglykolether (36 Mol EO) | 5 % | - | - |
| Tributylphenol-plyethylenglykolether (30 Mol EO) | - | 12 % | 4 % |
| Cyclohexanon | - | 15 % | 20 % |
| Xylolgemisch | 65 % | 25 % | 20 % |

Aus solchen Konzentrationen können durch Verdünnung mit Wasser Emulsionen jeder gewünschten Konzentration hergestellt werden.

| 2. Lösungen | a) | b) | c) | d) |
|---|---|---|---|---|
| Wirkstoffmischung | 80% | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxidiertes Kokosnußöl | - | - | 1 % | 5 % |
| Benzin (Siedegrenzen 160-190°) | - | - | 94% | - |

Die Lösungen sind zur Anwendung in Form kleinster Tropfen geeignet.

| 3. Granulate | a) | b) |
|---|---|---|
| Wirkstoffmischung | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschließend im Vakuum abgedampft.

| 4. Stäubemittel | a) | b) |
|---|---|---|
| Wirkstoffmischung | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel II oder Mischungen derselben mit einem Herbizid der Formel I (% Gewichtsprozent)

| 5. Spritzpulver | a) | b) | c) |
|---|---|---|---|
| Wirkstoffmischung | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mole EO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsion-Konzentrate | |
|---|---|
| Wirkstoffmischung | 10 % |
| Octylphenolpolyethylenglykolether (4-5 Mol EO) | 3 % |
| Ca-Dodecylbenzolsulfonat | 3 % |
| Ricinusölpolyglykolether (35 Mol EO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoffmischung | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

| 8. Extruder-Granulate | |
|---|---|
| Wirkstoffmischung | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschließend im Luftstrom getrocknet.

| 9. Umhüllungs-Granulate | |
|---|---|
| Wirkstoffmischung | 3 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmäßig aufgetragen. Auf diese Weise erhält man staubfreie Umhüllungs-Granulate.

| 10. Suspensions-Konzentrate | |
|---|---|
| Wirkstoffmischung | 40 % |
| Ethylenglykol | 10 % |
| Nonylphenolpolyethylenglykolether (15 Mol EO) | 6 % |
| Na-Ligninsulfonat | 10 % |
| Carboxymethylcellulose | 1 % |
| 37%ige wäßrige Formaldehyd-Lösung | 0,2 % |
| Silikonöl in Form einer 75%igen wäßrigen Emulsion | 0,8 % |
| Wasser | 32 % |

Der feingemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Die Fähigkeit der Verbindungen der Formel II, Kulturpflanzen vor der phytotoxischen Wirkung starker Herbizide zu schützen, kann aus den folgenden Beispielen ersehen werden.

Beispiel B1: Post-emergente phytotoxische Wirkungen des Herbizides N-[2-((3-Oxetanyl)-oxycarbonyl)-phenylsulfonyl]-N'-(4-methoxy-6-methylpyrimidin-2-yl)-harnstoff (Verb.Nr. 1.02) und der Mischungen Herbizid mit Safener der Formel II auf Weizen und Gerste

Unter Gewächshausbedingungen werden Weizen und Gerste in Kunststofftöpfen bis zum 4-Blattstadium angezogen. In diesem Stadium werden zum einen das Herbizid N-[2-((3-Oxetanyl)-oxycarbonyl)-phenylsulfonyl]-N'-(4-methoxy-6-methylpyrimidin-2-yl)-harnstoff (Verb.Nr. 1.02) alleine als auch die Mischungen des Herbizids mit den als Safener der Formel II zu prüfenden Substanzen auf die Testpflanzen appliziert. Die Applikation erfolgt als wässrige Suspension, hergestellt aus einem Suspensionskonzentrat (Formulierungsbeispiel 10), der Prüfsubstanzen mit 500 l Wasser/ha. Die Aufwandmengen betragen 15 g/ha für das Herbizid und 250 g/ha für die als Safener zu prüfenden Verbindungen der Formel II. 22 Tage nach Applikation wird mit einer Prozentskala ausgewertet. 100 % bedeutet Testpflanze ist abgestorben, 0 % bedeutet keine phytotoxische Wirkung. Es werden die in Tabelle B1 dargestellten Resultate erhalten. Die Resultate zeigen, dass mit den Safener in der Tabelle 4 die durch das Herbizid verursachte Schädigung auf Weizen und Gerste deutlich reduziert werden kann.

Die gleichen Resultate werden erhalten, wenn man die genannte wässrige Suspension aus einem Emulsions-Konzentrat (Formulierungsbeispiel 1), einer Lösung (Formulierungsbeispiel 2), einem Granulat (Formulierungsbeispiel 3), Stäubemittel (Formulierungsbeispiele 4 und 7), Spritzpulver (Formulierungsbeispiel 5), Emulsions-Konzentrat (Formulierungsbeispiel 6) oder einem Extruder- oder Umhüllungs-Granulat (Formulierungsbeispiele 8 und 9) herstellt.

Tabelle B1: Post-ermergente phytotoxische Wirkungen des Herbizides N-[2-((3-Oxetanyl)-oxycarbonyl)-phenylsulfonyl]-N'-(4-methoxy-6-methylpyrimidin-2-yl)-harnstoff (Verb.Nr. 1.02; 15 g/ha) und der Mischungen Herbizid mit Safener (Formel II; 250 g/ha) auf Weizen und Gerste.

| Herbizid Verb.Nr. | Safener Verb.Nr. | Phytotoxische Wirkung in % | |
|---|---|---|---|
| | | Weizen | Gerste |
| 1.02 | - | 75 | 75 |
| 1.02 | 4.01 | 10 | 45 |
| 1.02 | 4.02 | 25 | 45 |
| 1.02 | 4.03 | 10 | 55 |
| 1.02 | 4.04 | 15 | 65 |
| 1.02 | 4.05 | 15 | 50 |
| 1.02 | 4.06 | 60 | 65 |
| 1.02 | 4.07 | 20 | 70 |
| 1.02 | 4.08 | 10 | 60 |
| 1.02 | 4.09 | 20 | 55 |
| 1.02 | 4.10 | 35 | 65 |
| 1.02 | 4.11 | 45 | 75 |
| 1.02 | 4.12 | 5 | 40 |
| 1.02 | 4.13 | 30 | 50 |
| 1.02 | 4.14 | 20 | 65 |
| 1.02 | 4.15 | 15 | 65 |
| 1.02 | 4.16 | 10 | 60 |
| 1.02 | 4.17 | 10 | 45 |
| 1.02 | 4.18 | 10 | 45 |
| 1.02 | 4.19 | 10 | 45 |

Beispiel B2: Post-emergente phytotoxische Wirkungen verschiedener herbizider Sulfonylharnstoffe aus den Tabellen 1, 2 und 3 allein und in Mischungen mit dem Safener 1-(2-Chlorphenyl)-3-methoxycarbonyl-5-phenylpyrazol (Verb.Nr. 4.01)

Unter Gewächshausbedingungen werden Weizen und Gerste in Kunststofftöpfen bis zum 4-Blattstadium angezogen. In diesem Stadium werden einerseits die in Tabelle B2 aufgeführten Herbizide alleine als auch die Mischungen der Herbizide mit dem Safener 1-(2-Chlorphenyl)-3-methoxycarbonyl-5-phenylpyrazol (Verb.Nr. 4.01) auf die Testpflanzen appliziert. Die Applikation erfolgt als wässrige Suspension, hergestellt aus einem Suspensionskonzentrat (Formulierungsbeispiel 10), der Prüfsubstanzen mit 500 l Wasser/ha. Die Aufwandmenge des Safeners (Verb. 4.01) beträgt immer 125 g/ha, die Aufwandmengen der Herbizide sind aus Tabelle B2 ersichtlich. 18 Tage nach Applikation wird mit einer Prozentskala ausgewertet: 100 % bedeutet Testpflanze ist abgestorben, 0 % bedeutet keine phytotoxische Wirkung. Es werden die in Tabelle B2 dargestellten Resultate erhalten. Die Resultate zeigen, dass mit dem Safener (Verb.Nr. 4.01) die Kulturpflanzen Weizen und Gerste gegen die phytotoxische Wirkung der herbiziden Prüfsubstanzen geschützt werden können.

Die gleichen Resultate werden erhalten, wenn man die genannte wässrige Suspension aus einem Emulsions-Konzentrat (Formulierungsbeispiel 1), einer Lösung (Formulierungsbeispiel 2), einem Granulat (Formulierungsbeispiel 3), Stäubemittel (Formulierungsbeispiele 4 und 7), Spritzpulver (Formulierungsbeispiel 5), Emulsions-Konzentrat (Formulierungsbeispiel 6) oder einem Extruder- oder Umhüllungs-Granulat (Formulierungsbeispiele 8 und 9) herstellt.

Tabelle B2: Post-emergente phytotoxische Wirkungen verschiedener herbizider Sulfonylharnstoffe aus den Tabellen 1, 2 und 3 allein und in Mischungen mit dem Safener 1-(2-Chlorphenyl)-3-methoxycarbonyl-5-phenylpyrazol (Verb.Nr. 4.01; 125 g/ha).

| Herbizid Verb.Nr. | g/ha | Safener Verb.Nr. | Phytotoxische Wirkung in % Weizen | Gerste |
|---|---|---|---|---|
| 1.01 | 30 | ___ | 85 | 85 |
| | 15 | ___ | 40 | 70 |
| | 8 | ___ | 10 | 65 |
| 1.01 | 30 | 4.01 | 55 | 65 |
| | 15 | 4.01 | 25 | 50 |
| | 8 | 4.01 | 5 | 40 |
| 1.02 | 30 | ___ | 65 | 85 |
| | 15 | ___ | 45 | 75 |
| | 8 | ___ | 20 | 65 |
| 1.02 | 30 | 4.01 | 25 | 70 |
| | 15 | 4.01 | 5 | 55 |
| | 8 | 4.01 | 0 | 20 |
| 1.03 | 125 | ___ | 60 | 85 |
| | 60 | ___ | 30 | 75 |
| | 30 | ___ | 10 | 60 |
| 1.03 | 125 | 4.01 | 25 | 50 |
| | 60 | 4.01 | 5 | 30 |
| | 30 | 4.01 | 0 | 10 |

| Herbizid Verb.Nr. | g/ha | Safener Verb.Nr. | Phytotoxische Wirkung in % Weizen | Gerste |
|---|---|---|---|---|
| 1.04 | 30 | ___ | 35 | 70 |
| | 15 | ___ | 10 | 60 |
| | 8 | ___ | 0 | 30 |
| 1.04 | 30 | 4.01 | 0 | 25 |
| | 15 | 4.01 | 0 | 10 |
| | 8 | 4.01 | 0 | 0 |
| 1.05 | 125 | ___ | 90 | 90 |
| | 60 | ___ | 75 | 85 |
| | 30 | ___ | 60 | 75 |
| 1.05 | 125 | 4.01 | 70 | 80 |
| | 60 | 4.01 | 45 | 70 |
| | 30 | 4.01 | 15 | 60 |
| 1.06 | 500 | ___ | ___ | 40 |
| | 250 | ___ | ___ | 25 |
| | 125 | ___ | ___ | 15 |
| 1.06 | 500 | 4.01 | ___ | 10 |
| | 250 | 4.01 | ___ | 5 |
| | 125 | 4.01 | ___ | 0 |
| 1.07 | 500 | ___ | ___ | 20 |
| | 250 | ___ | ___ | 10 |
| | 125 | ___ | ___ | 5 |
| 1.07 | 500 | 4.01 | ___ | 5 |
| | 250 | 4.01 | ___ | 0 |
| | 125 | 4.01 | ___ | 0 |

| Herbizid Verb.Nr. | g/ha | Safener Verb.Nr. | Phytotoxische Wirkung in % Weizen | Gerste |
|---|---|---|---|---|
| 1.16 | 125 | --- | --- | 80 |
| | 60 | --- | --- | 40 |
| | 30 | --- | --- | 20 |
| | 15 | --- | --- | 10 |
| 1.16 | 125 | 4.01 | --- | 20 |
| | 60 | 4.01 | --- | 15 |
| | 30 | 4.01 | --- | 5 |
| | 15 | 4.01 | --- | 0 |
| 2.01 | 125 | ___ | 40 | 60 |
| | 60 | ___ | 20 | 30 |
| | 30 | ___ | 5 | 10 |
| 2.01 | 125 | 4.01 | 15 | 25 |
| | 60 | 4.01 | 5 | 15 |
| | 30 | 4.01 | 0 | 5 |
| 2.02 | 500 | ___ | 60 | 40 |
| | 250 | ___ | 15 | 20 |
| | 125 | ___ | 5 | 10 |
| 2.02 | 500 | 4.01 | 15 | 25 |
| | 250 | 4.01 | 5 | 10 |
| | 125 | 4.01 | 0 | 5 |
| 2.03 | 30 | ___ | 65 | 70 |
| | 15 | ___ | 25 | 40 |
| | 8 | ___ | 5 | 15 |
| 2.03 | 30 | 4.01 | 30 | 40 |
| | 15 | 4.01 | 10 | 25 |
| | 8 | 4.01 | 0 | 5 |

| Herbizid Verb.Nr. | g/ha | Safener Verb.Nr. | Phytotoxische Wirkung in % Weizen | Gerste |
|---|---|---|---|---|
| 2.04 | 30 | — | 25 | 65 |
| | 15 | — | 5 | 60 |
| | 8 | — | 0 | 50 |
| 2.04 | 30 | 4.01 | 0 | 30 |
| | 15 | 4.01 | 0 | 10 |
| | 8 | 4.01 | 0 | 5 |
| 2.05 | 30 | — | 75 | 90 |
| | 15 | — | 10 | 75 |
| | 8 | — | 0 | 60 |
| 2.05 | 30 | 4.01 | 20 | 65 |
| | 15 | 4.01 | 5 | 25 |
| | 8 | 4.01 | 0 | 15 |
| 2.06 | 125 | — | 50 | 55 |
| | 60 | — | 10 | 25 |
| | 30 | — | 5 | 15 |
| 2.06 | 125 | 4.01 | 10 | 30 |
| | 60 | 4.01 | 5 | 10 |
| | 30 | 4.01 | 0 | 5 |
| 2.08 | 250 | — | 85 | 75 |
| | 125 | — | 60 | 70 |
| | 60 | — | 25 | 60 |
| 2.08 | 250 | 4.01 | 60 | 45 |
| | 125 | 4.01 | 25 | 30 |
| | 60 | 4.01 | 5 | 25 |

| Herbizid Verb.Nr. | g/ha | Safener Verb.Nr. | Phytotoxische Wirkung in % Weizen | Gerste |
|---|---|---|---|---|
| 2.09 | 30 | ___ | 30 | 50 |
| | 15 | ___ | 10 | 15 |
| | 8 | ___ | 5 | 5 |
| 2.09 | 30 | 4.01 | 20 | 20 |
| | 15 | 4.01 | 5 | 5 |
| | 8 | 4.01 | 0 | 0 |
| 2.10 | 30 | ___ | 90 | 90 |
| | 15 | ___ | 60 | 80 |
| | 8 | ___ | 15 | 60 |
| 2.10 | 30 | 4.01 | 90 | 85 |
| | 15 | 4.01 | 35 | 65 |
| | 8 | 4.01 | 0 | 40 |
| 3.01 | 30 | ___ | 90 | 70 |
| | 15 | ___ | 60 | 50 |
| | 8 | ___ | 10 | 30 |
| 3.01 | 30 | 4.01 | 80 | 40 |
| | 15 | 4.01 | 40 | 30 |
| | 8 | 4.01 | 0 | 10 |

**Patentansprüche**

1. Mittel zur selektiven Kontrolle von Unkräutern in Kulturen von Nutzpflanzen, dadurch gekennzeichnet, daß es neben inerten Trägerstoffen und Zusatzmaterialien als Wirkstoff eine Mischung enthält, die
   a) aus einer herbizid wirksamen Menge eines Sulfonylharnstoffs der Formel I

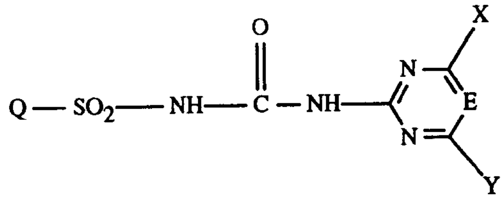

(I),

worin
Q einen Rest

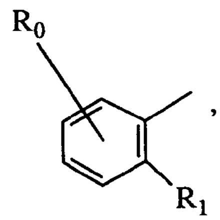
,
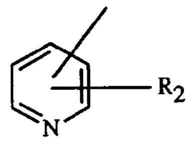

oder

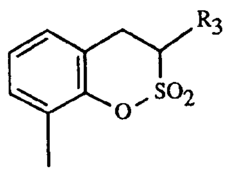
;

$R_0$ Wasserstoff, Halogen, Methyl oder Methoxy;
$R_1$ Halogen, $C_1$-$C_3$-Halogenalkyl, $C_1$-$C_3$-Halogenalkoxy, $C_1$- oder $C_2$-Alkoxy-$C_1$-$C_3$-alkoxy, $C_3$-$C_6$-Alkenyloxy, $C_3$-$C_6$-Alkinyloxy, $C_2$- oder $C_3$-Alkinyl,

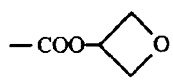

oder einen Rest

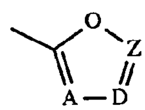

bedeutet;
A und Z unabhängig voneinander Stickstoff oder Methin sind;
D Stickstoff, Methin oder Methylmethin;
$R_2$ $C_1$-$C_3$-Halogenalkoxy, $C_1$- oder $C_2$-Alkoxy-$C_1$-$C_3$-alkoxy, $C_4$-$C_6$-Cycloalkyloxy, $C_3$-$C_6$-Cycloalkyl-$C_1$-$C_3$-alkoxy, -$COOR_4$ oder -$NR_5R_6$ ist;
$R_4$ $C_1$-$C_3$-Alkyl oder 3-Oxetanyl;
$R_5$ Wasserstoff oder $C_1$-$C_4$-Alkyl;
$R_6$ Wasserstoff, $C_1$-$C_4$-Alkyl oder -$COR_{11}$;
$R_{11}$ Wasserstoff oder $C_1$-$C_6$-Alkyl;
$R_3$ Wasserstoff oder Methyl;
X Halogen, Methyl, Ethyl, Methoxy, Ethoxy, $C_1$- oder $C_2$-Halogenalkoxy, Cyclopropyl, -$NHCH_3$ oder -$N(CH_3)_2$;
Y Methyl, Ethyl, Methoxy, Ethoxy, $C_1$- oder $C_2$-Halogenalkoxy oder Cyclopropyl; und
E Stickstoff oder die Methingruppe, bedeutet;
sowie die agrochemisch verträglichen Salze dieser Verbindungen;
wobei D und Z nicht gleichzeitig Stickstoff sein dürfen; und E die Methingruppe bedeutet, wenn X Halogen oder Difluormethoxy ist; und
b) als Safener aus einer herbizid-antagonistisch wirksamen Menge eines 1,5-Diphenylpyrazol-3-carbonsäure-Derivates der Formel II

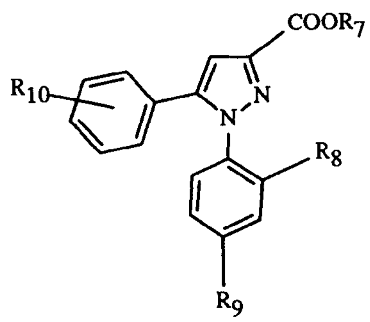 (II),

worin
$R_7$ Wasserstoff, $C_1$-$C_8$-Alkyl, $C_3$-$C_6$-Alkenyl, $C_3$-$C_6$-Cycloalkyl-$C_1$-$C_3$-alkyl, oder ein Alkalimetall oder Ammoniumkation ist; und
$R_8$, $R_9$ und $R_{10}$ unabhängig voneinander Wasserstoff oder Halogen bedeuten, besteht.

2. Mittel gemäß Anspruch 1, dadurch gekennzeichnet, daß es als Herbizid eine Verbindung der Formel Ia

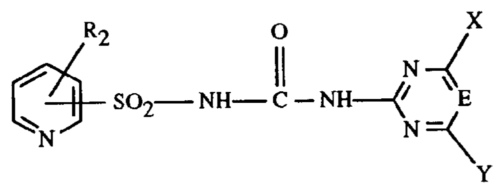 (Ia)

enthält, worin $R_2$, X, Y und E die in Anspruch 1 angegebenen Bedeutungen haben.

3. Mittel gemäß einem der Ansprüche 1 oder 2, dadurch gekennzeichnet, daß es als Herbizid eine Verbindung der Formel Ib

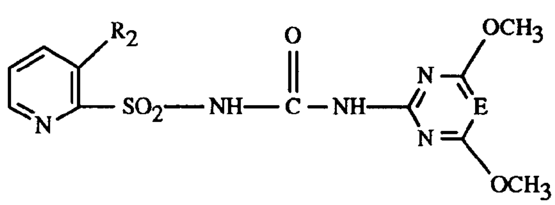 (Ib)

enthält, worin $R_2$ und E die in Anspruch 1 angegebenen Bedeutungen haben.

4. Mittel gemäß Anspruch 1, dadurch gekennzeichnet, daß es als Herbizid eine Verbindung der Formel Ic

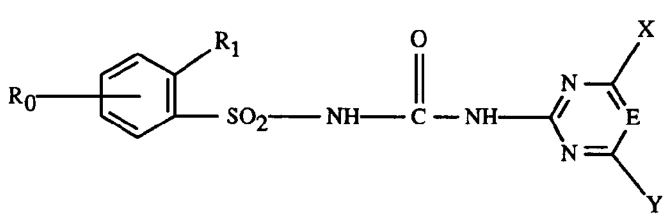 (Ic)

enthält, worin $R_0$, $R_1$, X, Y und E die in Anspruch 1 angegebenen Bedeutungen haben.

5. Mittel gemäß einem der Ansprüche 1 oder 4, dadurch gekennzeichnet, daß es als Herbizid eine Verbindung der Formel Id

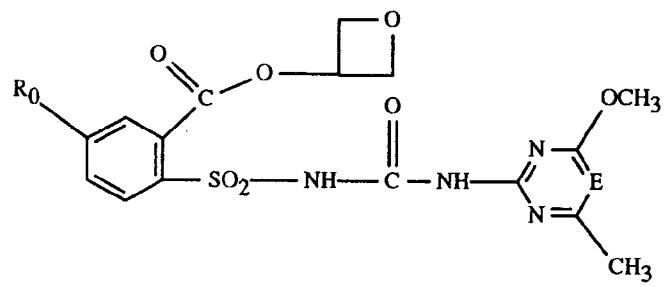

(Id)

enthält, worin E die in Anspruch 1 angegebene Bedeutung hat, und $R_0$ Wasserstoff oder Fluor bedeutet.

6. Mittel gemäss Anspruch 1, dadurch gekennzeichnet, dass es als Herbizid eine Verbindung der Formel I enthält, worin Q
einen Rest

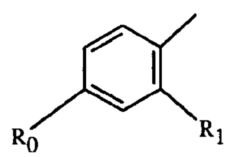 , 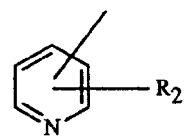

oder

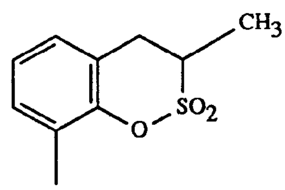 ;

$R_0$ Wasserstoff oder Halogen, insbesondere Fluor,
$R_1$ $C_1$-$C_3$-Monofluoralkyl, Difluormethoxy, Propargyloxy, Ethinyl,

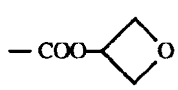

oder
einen Rest

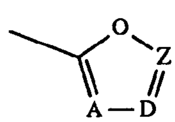

bedeutet; A und Z unabhängig voneinander Stickstoff oder Methin sind; D Stickstoff, Methin oder Methylmethin; $R_2$ in 2- oder 3-Position, alternierend mit der Position der $-SO_2NHCO$-Gruppe, $C_1$- oder $C_2$-Halogenalkoxy, Cyclopropylmethylenoxy, $COOR_4$ oder $NR_5R_5$; $R_4$ Methyl oder 3-Oxetanyl; $R_5$ Wasserstoff, Methyl oder Ethyl; $R_6$ Wasserstoff, Methyl, Ethyl, -CHO, $-COCH_3$ oder $-COC_2H_5$ ist; X und Y unabhängig voneinander Methyl, Methoxy, Ethoxy oder Cyclopropyl sind; und E Stickstoff oder die Methingruppe ist.

7. Mittel gemäß einem der Ansprüche 1 bis 6, dadurch gekennzeichnet, daß es als Safener ein 1,5-Diphenylpyrazol-3-carbonsäure-Derivat der Formel IIa

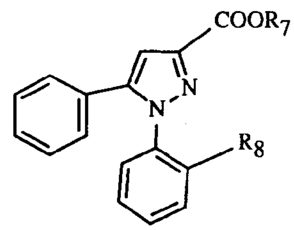

(IIa)

enthält.

8. Mittel gemäß einem der Ansprüche 1 bis 7, dadurch gekennzeichnet, daß es als Safener ein 1,5-Diphenylpyrazol-3-carbonsäureester der Formel IIb

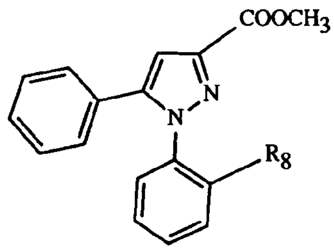

(IIb)

enthält.

9. Mittel gemäss Anspruch 1, dadurch gekennzeichnet, dass es als Safener eine Verbindung der Formel II enthält, worin $R_7$ Wasserstoff, $C_1$-$C_8$-Alkyl, Methallyl, Cyclohexyl-methylenyl, Natrium, Triethylammonium oder iso-Propylammonium; $R_8$ Wasserstoff oder Halogen; $R_9$ Wasserstoff oder Chlor; und $R_{10}$ Wasserstoff, Fluor oder Chlor, bedeuten.

10. Mittel gemäss einem der Ansprüche 1 bis 6 oder 8, dadurch gekennzeichnet, dass es als Safener die Verbindung der Formel IIc

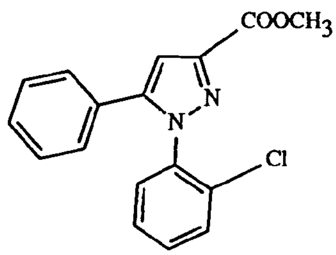

(IIc)

enthält.

11. Mittel gemäss Anspruch 1, dadurch gekennzeichnet, dass es als Herbizid N- [2-((3-Oxetanyl)-oxycarbonyl)-phenylsulfonyl]-N'-(4-methyl-6-methoxypyrimidin-2-yl)-harnstoff und als Safener eine Verbindung ausgewählt aus:
1-(2-Chlorphenyl)-3-methoxycarbonyl-5-phenylpyrazol;
1-(2,4-Dichlorphenyl)-3-methoxycarbonyl-5-phenylpyrazol;
1-(2-Chlorphenyl)-3-benzyloxycarbonyl-5-phenylpyrazol;
1-(2-Chlorphenyl)-3-methoxycarbonyl-5-(2,4-dichlorphenyl)-pyrazol; oder
1-(2-Chlorphenyl)-3-methoxycarbonyl-5-(2-fluorphenyl)-pyrazol;
enthält.

12. Mittel gemäss Anspruch 1, dadurch gekennzeichnet, dass es als Safener 1-(2-Chlorphenyl)-3-methoxycarbonyl-5-phenylpyrazol, und als Herbizid eine Verbindung ausgewählt aus:
N-[2-(Cyclopropylmethylenoxy)-3-pyridylsulfonyl]-N'-(4,6-dimethoxypyrimidin-2-yl)-harnstoff;

N-[3-(Cyclopropylmethylenoxy)-2-pyridylsulfonyl]-N'-(4,6-dimethoxypyrimidin-2-yl)-harnstoff;
N-[3-Dimethylamino-2-pyridylsulfonyl]-N'-(4,6-dimethoxypyrimidin-2-yl)-harnstoff;
N-[2-Dimethylamino-3-pyridylsulfonyl]-N'-(4-methyl-6-methoxypyrimidin-2-yl)-harnstoff:
N-[3-Dimethylamino-2-pyridylsulfonyl]-N'-(4-methyl-6-methoxypyrimidin-2-yl)-harnstoff;
N-[2-((3-Oxetanyl)-oxycarbonyl)-phenylsulfonyl]-N'-(4,6-dimethylpyrimidin-2-y1)-harnstoff;
N-[2-((3-Oxetanyl)-oxycarbonyl)-phenylsulfonyl]-N'-(4-methyl-6-methoxypyrimidin-2-yl)-harnstoff;
N- [2-((3-Oxetanyl)-oxycarbonyl)-phenylsulfonyl]-N'-(4,6-dimethoxypyrimidin-2-yl)-harnstoff;
N-[3-Difluormethoxy-2-pyridylsulfonyl]-N'-(4-methyl-6-methoxypyrimidin-2-yl)-harnstoff;
N-[3-((3-Oxetanyl)-oxycarbonyl)-2-pyridylsulfonyl]-N'-(4,6-dimethoxypyrimidin -2-yl)-harnstoff; oder
N-[2-((3-Oxetanyl)-oxycarbonyl)-4-fluor-phenylsulfonyl]-N'-(4-methyl-6-methoxypyrimidin-2-yl)-harnstoff;
enthält.

**13.** Mittel gemäss Anspruch 1 enthaltend als Wirkstoff 2 bis 95% einer Mischung aus Verbindungen der Formel II und der Formel I.

**14.** Verfahren zum selektiven Bekämpfen von Unkräutern und Gräsern in Nutzpflanzenkulturen, dadurch gekennzeichnet, daß man die Kulturen, deren Saatgut oder deren Anbaufläche gleichzeitig oder unabhängig voneinander mit einer wirksamen Menge eines Herbizides der Formel I gemäß einem der Ansprüche 1 bis 6 und einer herbizidantagonistisch wirksamen Menge eines 1,5-Diphenylpyrazol-3-carbonsäure-Derivates der Formel II gemäß einem der Ansprüche 1 oder 7 bis 10 behandelt.

**15.** Verfahren gemäß Anspruch 14, dadurch gekennzeichnet, daß man Kulturpflanzenbestände oder Anbauflächen für Kulturpflanzen mit 0,05 bis 2 kg/ha einer Verbindung der Formel I gemäß Anspruch 1 und einer Menge von 0,01 bis 0,5 kg/ha einer Verbindung der Formel II gemäß Anspruch 1 behandelt.

**16.** Verfahren gemäß Anspruch 14 zum selektiven Bekämpfen von Unkräutern und Gräsern in Getreidekulturen.

Europäisches Patentamt

# EUROPÄISCHER RECHERCHENBERICHT

Nummer der Anmeldung

EP 93 81 0095

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| P,X | EP-A-0 492 367 (HOECHST)<br>* Seite 3, Zeile 2 - Seite 7, Zeile 39 *<br>* Seite 8, Zeile 8 *<br>--- | 1-16 | A01N47/36<br>//(A01N47/36, 43:56, 25:32) |
| D,A | EP-A-0 268 554 (CIBA-GEIGY)<br>* Seite 2, Zeile 3 - Seite 7, Zeile 65 *<br>--- | 1-16 | |
| A | EP-A-0 147 365 (CIBA-GEIGY)<br>* Seite 1, Absatz 1 - Seite 6, Absatz 3 *<br>----- | 1-16 | |

RECHERCHIERTE SACHGEBIETE (Int. Cl.5 )

A01N

Der vorliegende Recherchenbericht wurde für alle Patentansprüche erstellt

| Recherchenort | Abschlußdatum der Recherche | Prüfer |
|---|---|---|
| DEN HAAG | 20 APRIL 1993 | LAMERS W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)